# EUROPEAN PATENT SPECIFICATION

(11) **EP 2 179 028 B1**
(45) Date of publication and mention of the grant of the patent: **13.08.2014**
(21) Application number: 08774380.3
(22) Date of filing: 26.06.2008
(51) Int. Cl.: C12N 1/20, C12P 21/06, A23L 1/30, A23L 1/305, A61K 35/74, A61K 38/04, C12R 1/01

(54) **A NOVEL STRAIN OF BIFIDOBACTERIUM AND ACTIVE PEPTIDES AGAINST ROTAVIRUS INFECTIONS**
NEUER BIFIDOBACTERIUM-STAMM UND AKTIVE PEPTIDE GEGEN ROTAVIRUSINFEKTIONEN
NOUVELLE SOUCHE DE BIFIDOBACTÉRIE ET PEPTIDES ACTIFS CONTRE DES INFECTIONS PAR UN ROTAVIRUS

(30) Priority: 27.06.2007 EP 07111189
(43) Date of publication of application: 28.04.2010
(60) Divisional application: 12180141.9
(73) Proprietor: Laboratorios Ordesa, S.l., 08830 Sant Boi de Llobregat (ES)
(72) Inventor: RIVERO URGELL, Montserrat, E-08830 Sant Boi de Llobregat (ES); FÀBREGA SÁNCHEZ, Joan, E-08006 Barcelona (ES); MORENO MUÑOZ, José Antonio, E-08222 Terrassa (ES); BATALLER LEIVA, Esther, E-46008 Valencia (ES); BUESA GÓMEZ, Javier, E-46010 Valencia (ES); GENOVÉS MARTÍNEZ, Salvador, E-46960 Aldaia (ES); RAMÓN VIDAL, Daniel, E-46183 L'Eliana (ES); VILLANUEVA ROIG, Adela, E-46210 Picaña (ES); CASINOS RAMO, Beatriz, E-46370 Chiva (ES)
(74) Representative: ZBM Patents - Zea, Barlocci & Markvardsen
(86) International application number: PCT/EP2008/058206
(87) International publication number: WO 2009/000899

(56) References cited:
- EP-A- 1 359 157
- EP-A- 1 571 213
- WO-A-01/10453
- WO-A-02/30958
- WO-A-02/074798
- BAE E-A ET AL: "PURIFICATION OF ROTAVIRUS INFECTION-INHIBITORY PROTEIN FROM BIFIDOBACTERIUM BREVE K-110" JOURNAL OF MICROBIOLOGY AND BIOTECHNOLOGY, KOREAN SOCIETY FOR APPLIED MICROBIOLOGY, SEOUL, KO, vol. 12, no. 4, August 2002 (2002-08), pages 553-556, XP009050414 ISSN: 1017-7825
- JANER C ET AL: "Enzymatic ability of Bifidobacterium animalis subsp lactis to hydrolyze milk proteins: Identification and characterization of endopeptidase O" APPLIED AND ENVIRONMENTAL MICROBIOLOGY, vol. 71, no. 12, December 2005 (2005-12), pages 8460-8465, XP002493851 ISSN: 0099-2240
- CLARE D A ET AL: "BIODEFENSE PROPERTIES OF MILK: THE ROLE OF ANTIMICROBIAL PROTEINS AND PEPTIDES" CURRENT PHARMACEUTICAL DESIGN, BENTHAM SCIENCE PUBLISHERS, SCHIPHOL, NL, vol. 9, no. 16, 2003, pages 1239-1255, XP008052495 ISSN: 1381-6128

## Description

The present invention relates to a novel strain of *Bifidobacterium* having anti-rotavirus activity. Thus the invention relates to the use of these agents for the treatment or prophylaxis of diarrhea produced by rotaviruses.

### BACKGROUND ART

Rotavirus belongs to the family *Reoviridae* and was given the name 'rota' because its double capsid looks like a wheel of a cart. Since it was discovered, rotavirus has been confirmed by many epidemiological studies performed worldwide to be a major cause of acute diarrhea in infants and young children. Rotaviruses are divided into 6 different antigenic groups (A-F). Rotaviruses of group A, B and C are found in both human and animals, and rotaviruses of group D, E and F are found in animals only. Rotaviruses of group A are largely distributed among human-derived strains, and subgroups are determined by its inner capsid protein VP6 and serological types are determined by the outside capsid proteins VP7 and VP4. Within group A, 4 subgroups have been distinguished. There are at least 14 G-serotypes, G1 to G14, based on the antigenicity of the VP7 protein, and 12 P-serotypes, based on the antigenic properties of the VP4 protein.

Rotavirus is known as the main cause of acute diarrhea and enteritis in children and in the young animals of cattle, horses, pigs and monkeys. The transmission of the infection occurs mainly by the fecal-oral route. Rotavirus causes frequently an acute watery diarrhea in children under 5 years of age.

This rotavirus-induced diarrhea is the result of perturbances in absorption of intestinal mucosal epithelial cells caused by the viral infection. Once the enterocytes of the small intestine become infected, the virus proliferates in their cytoplasms and causes an impairment in several transport systems (ions and/or solutes and water). Damaged cells suffer desquamation and spout out the virus into the intestinal lumen, so that the virus can be found in feces. When rotavirus replicates, damaged cells of villi are replaced by immature crypt cells not having absorption capability, causing malabsorption of sodium and glucose, which results in diarrhea. In addition, a non-structural rotavirus protein, NSP4, has been identified as a viral enterotoxin. NSP4 increases the intracellular calcium levels and affects the permeability of plasma membranes, causing an efflux of chloride, sodium and water, thereby inducing a secretory diarrhea.

The World Health Organization estimates that five million children under 5 years of age die of diarrhea every year, and 20% of them die of rotavirus induced diarrhea. Rotavirus induced diarrhea prevails in winter and is one of the leading cause of infant mortality in the developing countries. The best measure to prevent the disease is breast-feeding. Since mother's milk includes IgA acting against rotavirus, infantile diarrhea caused by rotavirus can be prevented by breast-feeding.

During the last few years research has focused on the potential use of probiotic agents. Probiotics are defined as live microorganisms which when administered in adequate amounts confer a health benefit on the host. The known benefits of enteral administration of probiotic microorganisms include enhanced host defense to disease improving the properties of the indigenous microbiota and increasing colonization resistance to the harmful microbiota. Probiotics have been suggested to play an important role in the formation or establishment of a well-balanced, indigenous, intestinal microbiota in newborn children or adults receiving high doses of antibiotics. Lactic acid bacteria, especially specific strains of *Lactobacillus* and species of *Streptococcus, Enterococcus* and *Bifidobacterium* genera have been recommended for their use as probiotics.

The bacterial genus *Lactobacillus* has been studied frequently in children with regard to its antidiarrheal properties since the 1960s. Studies published in the world literature have concluded that *Lactobacillus* is indeed safe and effective in treating and preventing infectious diarrhea, antibiotic-associated diarrhea, and diarrhea in children who are very susceptible as a result of poor nutrition, impaired immune status, or frequent exposure to pathogens. Despite these reports, physicians in the United States do not routinely recommend *Lactobacillus,* perhaps believing that its effectiveness has not yet been proven (cf. C.W. Christakis et al., "Lactobacillus therapy for acute infectious diarrhea in children: A meta-analysis" Pediatrics 2002, vol. 109, pp. 678-84). J.M. Saavedra et al., "Feeding of Bifidobacterium bifidum and Streptococcus thermophilus to infants in hospital for prevention of diarrhea and shedding of rotavirus" Lancet 1994, vol. 344, pp. 1046-9, assayed a formula with *Bifidobacterium bifidum* and *Streptococcus thermophilus* with hospitalized children.

A number of studies have shown the efficacy of other probiotic agents, including *Lactobacillus rhamnosus* GG (LGG), in the prevention of diarrhea. Several pediatric clinical trials showed the efficacy of LGG in the treatment of rotavirus gastroenteritis (cf. S. Guandalini et al., "Lactobacillus GG administered in oral rehydration solution to children with acute diarrhea: a multicenter European study", J. Pediatr. Gastroenterol. Nutr. 2000, vol. 30, pp. 54-60).

Other approaches have been proposed for treating diarrhea, for example:

EP 1571213 A1 discloses a new strain of *Bifidobacterium longum* AR81 (KCCM-10492), and an active protein separated from the strain with the sequence of 10 amino acids HLDLAVENT, which have rotavirus-inhibiting activity.

Examples and data depicted in EP 1571213 A1 are all performed with a cytoplasmic protein, a protease present within the cells. Thus, to obtain the active ingredient firstly the cells are to be processed (lysated) to obtain the cytoplasm components or the cell-wall components.

JP 08259597 discloses a protein prepared from the culture of *Bifidobacterium longum* strain SBT 2928 capable of interfering with the adhesion of pathogens (*Escherichia coli, Pseudomonas aeruginosa,* etc.) to asialo-GM1. The protein exhibits a molecular weight 10.4 KDa (gel filtration) or 52 KDa (SDS-PAGE) and pl 5.8. Its N-terminal amino acids are defined as VDPHAIVPSNFDFAFL.

EP 1353681 A1 describes the use of the strain *Bifidobacterium* CNCM-2168 to prevent infection of intestinal cells by rotaviruses.

WO 2003010297 A1 describes probiotic *Bifidobacferium* strains AH208, AH209, AH21 0, AH211, AH212 or AH214, and their use in the treatment of several diseases.

WO 0042168 A1 describes a strain of *Bifidobacterium* (*Bifidobacterium longum infantis* UCC35624) with significantly immunomodulatory effects, useful in the prophylaxis and/or treatment of undesirable inflammatory activity, especially gastrointestinal inflammatory activity such as inflammatory bowel disease or irritable bowel syndrome.

US 5922375 describes a strain of *Bifidobacterium longum* (JBL 28-1/3300) and its use against diarrhea.

On the other hand, document EP1359157 discloses casein-derived peptides, obtained by hydrolysis of casein by *Lactobacillus helveticus* and having the effect of inhibiting a metalloproteinase. The peptides are used in food products for inhibition of matrix-metalloproteinases, and therefore applied for inhibition of cancer invasion and metastasis, tissue ulceration, arthritis and periodontitis as well as inhibition of osteoclasts.

Document WO 0230958 discloses the decapeptide of sequence HQPHQPLPPT that, once isolated by enzymatic hydrolysis of bovine casein, influences the growth of an insect cell line (namely the insect cell line IPLB-Sf-21).

There is an urgent need, both in the developed world and in the developing countries, for products and methods that would be effective in treating infectious diarrhea, and specifically diarrhea caused by rotavirus infection or associated with antibiotic therapy.

### SUMMARY OF THE INVENTION

The problem to be solved by the present invention is the provision of agents with anti-rotavirus activity and thus, effective for the treatment of diarrhea.

During the extensive studies leading to the present invention the inventors have investigated different bacterial strains isolated from breast-fed infant feces. The different strains were examined for their capability to prevent infection of intestinal cells with rotaviruses that are known to cause diarrhea.

The solution is based on the *Bifidobacterium longum* biovar *infantis* strain deposited with the Accession Number CECT 7210.

As it is illustrated below in a non-restricted way, the inventors have found that the *Bifidobacterium longum* biovar *infantis* strain of the present invention is effective for the protection against rotavirus infection. The strain of the invention has proven particularly useful for the inhibition of replication of several rotavirus strains. The inhibitory effect is obtained with both the intact probiotic cells or with the supernatants of cultures of the strain grown in the presence of casein. The effect is observed with intact whole probiotic cells, not being necessary the lysis of the probiotic cells to see inhibitory effect. This makes the strain of the invention useful in compositions and products where the strain is in entire/living form. The strain of the invention is active against diarrhea infections due to the effect in the enhancement of the immune response and also to the presence of peptides in the supernatant resulting from the fermentation of the strain.

Thus, in one aspect the present invention provides a strain of *Bifidobacterium longum* biovar *infantis* deposited in the "Colección Española de Cultivos Tipo (CECT)" under the accession number CECT 7210, or a mutant thereof having an activity of inhibiting rotavirus infection an having the ability to produce a peptide from the fermentation of cow milk β-casein, said peptide having a length from 11 to 17 amino acids and an amino acid sequence which comprises SEQ ID NO: 1. The strain CECT 7210 was deposited on 20 November 2006.

In a second aspect the present invention relates to a bacterial culture comprising the strain of the invention.

A third aspect of the invention relates to a bacterial preparation which comprises the strain of the invention.

A fourth aspect of the invention relates to a food product which comprises a nutritionally effective amount of the strain of the invention, together with appropriate amounts of other edible ingredients.

In a fifth aspect, the invention relates to a nutritional composition which comprises a nutritionally effective amount of the strain of the invention, together with appropriate amounts of other edible ingredients.

In a sixth aspect, the invention relates to a pharmaceutical composition which comprises a therapeutically effective amount of the strain of the invention, together with appropriate amounts of pharmaceutical acceptable excipients and/or carriers.

A seventh aspect of the invention relates to the strain as defined above, for its use as a probiotic.

Other aspects of the present invention relate to the use of the strain of the invention, for the manufacture of a medicament for the therapeutic and/or preventive treatment of diarrhea in an animal including a human, or for the manufacture of a food product, or of a nutritional composition.

Another aspect relates to the use of the strain of the invention, for the preparation of a peptide having a length from 11 to 17 amino acids and an amino acid sequence which comprises SEQ ID NO: 1, or a pharmaceutically acceptable salt thereof.

The invention may alternatively be formulated as a method for therapeutic and/or preventive treatment of diarrhea, in an animal including a human, comprising administering to said animal in need thereof an effective amount of the strain of the invention. The treatment of diarrhea is extended to diarrhea caused by gastrointestinal virus such as norovirus, rotavirus and astrovirus, or by microbial pathogens such as members of the genera *Escherichia, Clostridium, Salmonella, Shigella* and *Vibrio.* The treatment of the invention is particularly useful for diarrhea caused by rotavirus infection.

Finally, the invention relates to a process for the preparation of a peptide as defined above, or a pharmaceutically acceptable salt thereof, the process comprising fermenting cow-milk β-casein which comprises the amino acid sequence SEQ ID NO: 1 with the strain of the invention, and optionally isolating the peptide and converting them in a pharmaceutically acceptable salt.

These and other objects of the present invention will be further described in the detailed description section that follows, and they are not intended to be limiting of the present invention. Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention.

### DETAILED DESCRIPTION OF PARTICULAR EMBODIMENTS

The detailed characterization of the strain of the invention and the peptides isolated from the supernatant of the strain after fermentation, and their anti-rotavirus activity, are described in sections further on.

As it is used in the art, the term "microorganism" refers to organisms that are microscopic, and include bacteria, yeasts, fungi, archaea or protists. The term "bacteria" is used herein as in the art, to designate an unicellular microorganism which is prokaryotic, i.e., that lack a membrane-bound nucleus and organelles. The term "probiotic" refers to bacteria in the context of dietary supplements. Generally, the term probiotic refers to dietary supplements containing potentially beneficial bacteria or yeast, with lactic acid bacteria as the most common microbes used. The term "effective amount" as used herein, means an amount of an active ingredient high enough to deliver the desired benefit, but low enough to avoid serious side effects within the scope of medical judgment. The term "pharmaceutically acceptable" as used herein pertains to compounds, materials, compositions, and/or dosage forms which are, within the scope of sound medical judgment, suitable for use in contact with the tissues of a subject (e.g. human) without excessive toxicity, irritation, allergic response, or other problem or complication, commensurate with a reasonable benefit/risk ratio. Each carrier, excipient, etc. must also be "acceptable" in the sense of being compatible with the other ingredients of the formulation. Suitable carriers, excipients, etc. can be found in standard pharmaceutical texts, for example, Remington's Pharmaceutical Sciences, 18th edition, Mack Publishing Company, Easton, Pa., 1990.

The scope of the present invention also encompasses bacteria obtained by mutation of the strain *Bifidobacterium longum* biovar *infantis* CECT 7210, provided that the resulting bacteria have the activity of inhibiting rotavirus. In particular embodiments of the invention, the mutant is a genetically modified mutant obtained by classical mutagenesis (i.e. using chemical or physical agents) or by genetic engineering techniques.

The general use of the strain of the invention is in the form of viable cells. This means the introduction of the living bacteria in an unfermented composition. In this way, the administration of such compositions allows the anti-rotavirus activity to take place in the individual gastrointestinal tract.

However, the invention can also be extended to non-viable cells in compositions comprising beneficial factors expressed by the strain in a previous process. In partiular, in the present invention, this may be the result of exposing the strain of the invention to fermentation in a suitable medium so that the peptide produced by the strain of the invention can be produced. Suitable media for the production of the peptide produced by the strain of the invention is cow milk based media comprising β-casein but others media composition comprising cow milk β-casein are also effective in the production of the peptide. Thus, the individual ingests the fermented product which comprises the peptide produced by the strain of the invention and the non-viable cells resulting from the fermentation process. Compositions can comprise a combination of non-viable and viable cells.

In this regard, one embodiment of the present invention is a bacterial preparation comprising the strain of the invention in the form of pills, capsules or freeze-dried powder to be administered directly.

In other embodiments, the strain of the invention may be used for the preparation of a variety of food products, such as a milk products, a yogurt, a curd, a cheese (e.g. quark, cream, processed, soft and hard), a fermented milk, a milk powder, a milk based fermented product, an ice-cream, a fermented cereal based product, a milk based powder, a beverage, a dressing, and a pet food. Examples of other food products are meat products (e.g. liver paste, frankfurter and salami sausages or meat spreads), chocolate spreads, fillings (e.g. truffle, cream) and frostings, chocolate, confectionery (e.g. caramel, fondants or toffee), baked goods (cakes, pastries), sauces and soups, fruit juices and coffee whiteners. However, the term "food product" is used herein in its broadest meaning, including any type of product, in any form of presentation, which can be ingested by an animal.

In other embodiments, the strain of the invention may be used for the preparation of a variety of nutritional compositions. Particular embodiments are a dietary supplement, an additive, and an infant formula. Dietary supplements intend to supply nutrients, (vitamins, minerals, fatty acids or amino acids) that are missing or not consumed in sufficient quantity in a person's diet (infants, pregnant women, elderly people, etc). In a particular embodiment, the strain of the invention is homogenized with other ingredients, such as cereals or powdered milk to constitute an infant formula.

In such products and compositions, the strain is present in an amount from about 10⁵ cfu/g to about 10⁹ cfu/g of the composition, and preferably in an amount of 10⁷ cfu/g, according to the current legislation. For the purpose of the present invention the abbreviation "cfu" shall designate a "colony forming unit" that is defined as number of bacterial cells as revealed by microbiological counts on agar plates. Depending on the product, bacteria will be living or non-viable.

The present invention also provides pharmaceutical compositions comprising the strain of the invention. In this regard, the pharmaceutical composition may be prepared in form of tablets, dried oral supplements, dry tube feeding, etc., with the amount of bacteria to be incorporated therein being in the range of 10⁷ cfu/g to about 10¹¹ cfu/g of product, and preferably in an amount of 10⁹ cfu/g. Based upon the desired objective the person skilled in the art will select the appropriate excipients and/or carriers. *Bifidobacterium* are non stable in liquid form, so dried preparations are preferred.

In general, the compositions may comprise the bacteria of the invention as single probiotic agent against diarrhea, combinations of such probiotics or combinations with other therapeutic/nutraceutical agents depending on the condition.

In a particular embodiment, the peptide amino acid sequence produced by the strain of the invention is selected from the group consisting of SEQ ID NO: 1-16: In a more particular embodiment, the peptide has the amino acid sequence MHQPHQPLPPT (SEQ ID NO: 1) and a molecular weight of 1282.63 Da. In another embodiment, the peptide has 13 amino acids, a molecular weight of 1513 Da, and sequence MHQPHQPLPPTVM (SEQ ID NO: 3). These sequences correspond to fragments of cow milk β-casein Other peptides with an amino acid sequence comprising SEQ ID NO: 1 and produced by the strain of the invention can be useful for the purposes of the invention (SEQ ID NO: 2, 4-16).

The invention relates to a process fo the preparation of the peptides described above, comprising fermenting cow milk β-casein which comprises the amino acid sequence SEQ ID NO: 1 with a strain of the invention, and optionally isolating the peptide and/or converting them in a pharmaceutically acceptable salt.

The following sections describe the characterization of the strain of the invention, the characterization of the peptides isolated from the supernatant of the strain of the invention after fermentation, and the anti-rotavirus activity of the strain and the peptides.

### 1. Isolation of microorganisms

1 g of feces of breast fed infant was dissolved in PBS buffer (phosphate buffer pH 7.0) homogenizing the sample by means of maceration during 3 min in a stomacher (Bagmixer 400P, Interscience Worlwide, France). A 1 ml aliquot of the homogenate was incubated in Man Rogosa Sharpe broth (MRS) supplemented with 0.05% (w/v) of cysteine (MRS-C; Sigma, St. Louis, Mo.) to pH 3.0 with HCl during 17 h at 37 °C in anaerobiosis. Aliquots of this pre-incubation were seeded at different dilutions in a general medium MSR-C plates and in two selective mediums for *Bifidobacferium:* BSM (BSM, Fluka, Buchs, Switzerland) and BFM (BFM; Y. Nebra et al., "A new selective medium for Bifidobacterium spp." Applied and Enviromental Microbiology, 1999, vol. 65, pp. 5173-6) and were incubated during 36 to 72 h. Morphology of the colony and the type of cellular wall of the obtained strains were determined by Gram staining. With this protocol, bacteria resistant to acid pH were selected by means of the pre-incubation of the feces samples in liquid MRS-C during 17 h at pH 3.0. Of all the obtained strains, 30 isolates were selected to be seeded simultaneously in plates of *Lactobacillus* medium (MRS-C) and in selective media of *Bifidobacferium* (BSM and BFM). It was observed that a 70% belonged to the *Bifidobacferium* genus as opposed to 30% that belonged to the *Lactobacillus* genus.

Some strains were selected for characterization. The following results are for the selected strain of the invention, the *Bifidobacterium longue* biovar *infantis* deposited in the Colección Española de Cultivos Tipo (CECT) under the accession number CECT 7210.

### 2. Taxonomic characterization of CECT 7210 strain by sequencing

Almost full sequence of the 16S rRNA was amplified and sequenced using a ABI PRISM BigDye Terminator Cycle Sequencing Ready Reaction Kit. The DNA was checked for purity, using standard methods.

DNA templates were amplified by the polymerase chain reaction (PCR) on a thermocycler, using universal primers amplifying a 1000 bp region of the 16S rRNA gene, 616V: 5'-AGAGTTTGATYMTGGCTCAG-3' (SEQ ID NO: 17), 699R: 5'-RGGGTTGCGCTCGTT-3' (SEQ ID NO: 18). The amplification mixture (100 µl) comprised 2 µl (50 pmol/µl) each of 616V and 699R primers, 0.5 µl (2 U/µl) of Taq DNA Polymerase (Finnzymes), 10 µl of 10x reaction buffer (Finnzymes), 10 µl of dNTP mixture (containing 1 mM each of dATP, dGTP, dCTP and dTTP, Roche), 70 µl of sterile filtered water (Milli-Q purification system, Millipore) and 5.5 µl of DNA template. The DNA templates were amplified by initial denaturation at 94 °C for 10 min, followed by 40 cycles of denaturation at 94 °C for 1 min, annealing at 55 °C for 1 min, extension at 72 °C for 1 min, and a final extension at 72 °C for 10 min. Controls, devoid of DNA, were simultaneously included in the amplification process. The integrity of PCR products was assayed by the development of single bands following electrophoresis for 1 h at 100 V in 2% (w/v) agarose gels in Tris-borate EDTA buffer.

Amplicons were purified using a commercial kit and subsequent sequencing reactions were performed using the Big Dye Terminator v3.1 cycle sequencing kit, premixed format. Sequencing primers were the same ones used in the amplification reaction but diluted ten times (5 pmol). The resulting sequences were automatically aligned and then inspected by eye. The resulting 16S rRNA gene sequences were compared in a BLAST search with those in the NCBI.

The obtained sequence was as described in SEQ ID NO: 19 and correlates with the identification of the CECT 7210 strain as *Bifidobacterium longum* biovar *infantis.*

### 3. Physiological characterization of CECT 7210 strain for its use as probiotic

### 3.1. Resistance to gastrointestinal juices

Gastric and pancreatic juices were prepared immediately before their use, resuspending pepsin of pig stomach mucous membrane (3 g/l) and pancreatin of pig pancreas (1 g/l) in sterile saline solution 0.5% (w/v). The bacterial strain grew 24 h at 37 °C in anaerobiosis, it was washed twice in saline solution 0.5% and it was brought to an inoculum of 10⁸-10⁹ cfu/ml. The assay emulated conditions and time of exhibition to which bacteria are subjected in their passage by the gastric and intestinal tract in two stages. The first stage consists in the simulation of the passage by the gastric tract, for which it was prepared an inoculum of 1 % (v/v) of the bacteria in 10 ml of saline solution with pepsin adjusted to pH 3.0 with HCl and incubating at 37 °C in anaerobiosis during 120 min. The second stage consists in the simulation of the passage by the intestinal tract, for which the cells were neutralized washing them with a phosphate buffer (PBS) at pH 7.0 and they were resuspended in the saline solution with pancreatin adjusted to pH 8.0 with NaOH and incubating at 37 °C in anaerobiosis during 240 min. Samples were taken at 0/90/120 min in the first stage and at 0/60/240 min in second, making in each case serial dilutions, seeding 100 µl of each dilution per plate of solid medium MRS-C and incubating two days at 37 °C in anaerobiosis. As viability control throughout the process, the strain was inoculated in saline solution and it was incubated in the same conditions taking aliquots at the beginning, after 120 min and at the end of the assay.

After the incubation, the bacteria were recovered in MRS-C plates and viable counts were made after 48 h of incubation. The CECT 7210 strain showed a loss of viability after exposition to juices similar to that obtained by using other commercial probiotics. Percentage of viability was calculated as the relationship between the number of viable cells obtained in each one of the assay stages per the number of viable cells at time zero (inoculum 10⁷-10⁸ cfu/ml).

**TABLE 1. In vitro effect of the gastrointestinal juices in the viability of CECT 7210 strain.**

| | Pepsin pH 3, 120 min | | Pancreatin pH 8, 240 min | |
|---|---|---|---|---|
| Inoculum cfu/ml | count | % viable | Count | % viable |
| 1.6x10⁸ | 5.5x10⁶ | 3.4% | 3.1x10⁵ | 0.2% |

### 3.2. Profile of sensitivity to antibiotics

The sensitivity of the CECT 7210 strain to antibiotics was determined by means of techniques of diffusion in agar using discs impregnated with different antibiotic concentrations. The bacteria grow in liquid MRS-C at 37 °C during 24 h were harvested washing them several times with saline solution and adjusting the inoculum to 10⁹ cfu/ml (DO₆₀₀ = 5). In these conditions 100 µl of the cellular suspension was taken and seeded in 10 ml of semisolid agar MRS-C 0.7% (w/v) at 50 °C. This agar was spilled on a MRS-C plate and it was left solidifying at room temperature. Once dried, the antibiotic discs were placed on the surface. After 24 h of incubation in anaerobiosis, it was observed presence or absence of inhibition halos. A total of 16 antibiotics were tested to see the resistance or sensitivity of the strain (TABLE 2).

**TABLE 2. Resistance to antibiotics for CECT 7210 strain. Values expressed in mm of inhibition halo.**

| Antibiotic | CECT 7210 strain |
|---|---|
| Arnoxicillin (25 µg) | 25 |
| Carbenicillin (100 µg) | 32 |
| Oxacillin (1 µg) | 0 |
| Penicillin (6 µg) | 30 |
| Vancomycin (30 µg) | 0 |
| Chloramphenicol (30 µg) | 21 |
| Erythromycin (15 µg) | 26 |
| Gentamicin (10 µg) | 0 |
| Kanamycin (30 µg) | 0 |
| Tetracyclin (30 µg) | 30 |
| Metronidazole (5 µg) | 0 |
| Nalidixic acid (30 µg) | 0 |
| Rifampicin (5 µg) | 33 |
| Trimethoprim (5 µg) | 12 |
| Polymyxin B (300 IU) | 0 |
| Sulfonamide (200 µg) | 0 |

### 3.3. Resistance to biliary salts, NaCl and acid pH

The resistance of the CECT 7210 strain to different concentrations of biliary salts Oxgall, NaCl and pH was analyzed. The test is based on the monitorization of the bacteria's growth on plates of 96 wells. To each well 100 µl of and inoculum at OD₆₀₀ = 1 was added and 175 µl of MRS-C medium supplemented with 0%, 0.5%, 1%, 2% and 3% of Oxgall (w/v), MRS-C medium supplemented with 0%, 2%, 3%, 6%, 8% and 10% NaCl (w/v) or MRS-C at pH 6.2, pH 3.0, pH 2.0 and pH 1.5. The growth was analyzed at 655 nm in a microplates reader during 24 h of incubation at 37 °C in anaerobic conditions. The results showed that CECT 7210 strain was able to grow in presence of the different concentrations of biliary salts, no difference was found between the four concentrations tested. Regarding resistance to pH, the results showed that for the assayed acid pHs, an inhibition of the growth of CECT 7210 strain exists for pH lower than 3.0. These data serve as an indicator of the sensitivity of CECT 7210 strain to acidic pHs in an *in vitro* tests during long periods of time (24 h). Nevertheless, it is necessary to consider that *in vivo* conditions are others. On one hand, the transit time of the probiotic in the stomach, where these pHs are reached, does not surpass habitually two hours and it goes quickly to the thin intestine where pH is neutralized allowing the bacterial growth. On the other hand, *in vivo,* the probiotic is ingested together with other compounds (vehicle) that can act cushioning the inhibiting effect of the stomach pH. Regarding to NaCl resistance, the result showed that for concentrations between 0 and 2% a growth inhibition was not appreciated. However, once the concentration was up to 3-6%, a little growth inhibition was observed but is with 8-10% NaCl when the growth is apparently inhibited.

### 3.4. Adhesion of CECT 7210 strain to the intestinal mucus

Pig type III mucin was used at concentration 0.5 mg/ml. CECT 7210 strain was radioactively labeled adding to the culture medium 10 µl/ml of tritiated timidine (5-³H-timidine 120 Ci/mmol; Amersham Biosciences) and incubated at 37 °C during 17 h in anaerobiosis.Three comercial strains of *Lactobacillus casei* (LC), *Lactobacillus rhamnosus* (LR) and *Bifidobacterium longum* biovar *infantis* (BLI) were used as controls in the adhesion study. The mucus was dissolved in HEPES buffer (N-2-hydroxyethyl piperazine-N-2-ethansulphonic acid) - Hanks (HH; 10 mM HEPES, pH 7.4) at the desired concentration and was immobilized in polystyrene multiwell plates (Maxisorp, Nunc) adding 100 µl of this solution to each of the well and incubating at 4 °C overnight. The non-immobilized mucus was removed by means of two successive washings with 200 µl of HH buffer. After the last washing, 100 µl of HH buffer was added to each well. The radioactively labeled bacteria were centrifuged (6000 rpm during 7 min) and washed twice with HH buffer to eliminate the non-metabolized timidine, after which the concentration of bacteria was determined by means of the measurement of the absorbance at 600 nm adjusting to an OD of 0.25 ± 0.05 with the purpose of standardizing the number of bacteria to 10⁷-10⁸ cfu/ml. 100 µl of labeled cells were added to each of the wells where there was the mucus immobilized, and it was incubated 1 h at 37 °C. The elimination of the non-adhered bacteria was done by means of two washings of 200 µl with HH buffer. The bacteria adhered were released by means of a scraping of the immobilized mucus in each well and were later lysated with 1% (w/v) of SDS in 0.1 M NaOH (200 µl per well) incubating the plates at 60 °C during 1 h. The content of the cells was transferred to eppendorf tubes that contained scintillation liquid in order to measure the radioactivity in a scintillation counter. The adhesion was expressed as percentage of radioactivity recovered after the adhesion in relation to the radioactivity of the bacterial suspension added to the immobilized mucus. The bacterial adhesion was determined in three independent experiments and each assay was made by quadruplicated.

CECT 7210 strain showed a good adhesion capability of 9.9% similar to control strains (BLI 6.4%, LC 7.0% and LR 9.8%).

### 4. In vitro activity against rotavirus

### 4.1. Competition effect of the CECT 7210 strain vs. human rotavirus Wa against cellular binding sites.

CECT 7210 strain cells were grown during 17 h at 37 °C in anaerobiosis. The cultures were washed with saline solution (0.09%) to an inoculum of 3x10⁶ cfu/ml. Simultaneously, the formation of a monolayer of HT-29 and MA-104 cells was induced in 96-well plates. The assays were performed following two strategies A and B.

Strategy A - incubation of CECT 7210 strain with the rotavirus first, and later infection of cell cultures with the mixture virus-bacterial suspension Strategy B - incubation of CECT 7210 strain with the cell cultures first, and later infection with rotavirus.

Strategy A: 30 µl of the CECT 7210 strain bacterial suspension containing 3x10⁶ bacteria was mixed with 70 µl of D-MEM medium without serum. To this suspension 100 µl of human rotavirus Wa in a concentration 1x10⁷ ffu/ml ("ffu" is focus-forming units) were added, to the suitable dilution, in D-MEM medium. The mixtures virus-bacterial suspension, were incubated at 37 °C during 1 h. The monolayers of cells were washed 3 times with PBS buffer and were inoculated with the mixture virus-bacterial suspension during 18 h at 37 °C with 5% of CO₂. The cells infected with rotavirus were identified by means of the immunoperoxidase technique to evaluate the inhibitory capacity of the viral replication.

Strategy B: 30 µl of the CECT 7210 strain bacterial suspension containing 3x10⁶ bacteria was mixed with 70 µl of D-MEM medium without serum. The maintenance medium of the cellular monolayers was eliminated washing them 3 times with PBS buffer and 100 µl/well of the bacterial suspension were added. After one hour of incubation at 37 °C, 100 µl/well of virus (1x10⁷ ffu/ml) in D-MEM medium were added. Then, it was incubated during 18 h at 37 °C with 5% of CO₂ and the cells infected with rotavirus were identified also using the immunoperoxidase technique (*vide infra*).

In order to detect the viral antigen with immunoperoxidase in cultures of HT-29 and MA-104 cells, after infecting the cells cultures with the virus, these were washed twice with PBS buffer, fixed with methanol-acetone (1:1) during 15 min and washed again with PBS buffer. The monoclonal antibody anti-VP6 2F3E7 diluted 1/200 in PBS buffer containing 1% BSA (100 µl/well) was added, and was incubated at 37 °C during 1 h. After two washings with PBS buffer, 100 µl/well of mouse anti-IgG antibody conjugated to peroxidase (Sigma) diluted 1/2000 in PBS-BSA buffer 1% were added, and it was incubated at 37 °C during 1 h. After three washings with PBS buffer, 100 µl/well of diamine-benzidine substrate were added (6 mg DAB, 9 ml of 50 mM Tris-HCl pH 7.6 and 0.1 ml H₂O₂). Infectious foci stained with peroxidase were counted with an inverted microscope. The count of the number of infectious focus was made in 5 defined fields by well. The arithmetic average was calculated to determine the number of focus per microscopic field and they were compared with the data of a virus control without treatment with CECT 7210 strain to obtain the percentage of reduction of infectious foci.

HT-29 and MA-104 cell cultures were used and an inoculum of CECT 7210 strain bacteria of 3x10⁶ cfu/ml was added, in PBS buffer and a high dose of human rotavirus Wa. These assays were made following the two strategies explained above. The results obtained in both assays were similar. Considering the media of both assays, CECT 7210 strain produced a reduction of infectious foci of a 39.17% in HT29 cells and 25% in MA104 cells. However, a slightly increase of inhibition was detected in the assay B indicating that the interaction of the probiotic with the surface of the mammalian cell plays an important role in the mechanism of inhibition.

### 4.2.1. Production of supernatant containing the substance of interest

A fermentation of 10 liters was carried out with CECT 7210 strain in MRS medium supplemented with cysteine (0.005% w/v) in order to obtain a supernatant. The incubation was carried out at 37 °C during 17 hours without agitation and with anaerobiosis generator, being reached a final optical density value at 600 nm of 5 units. The amount of inoculum used was 1% of the volume of fermentation. The supernatant was subjected firstly to a neutralization process based on a centrifugation at 10000 rpm during 15 min and a neutralization with 10 N NaOH until reaching pH 6.5.After that, a concentration process by means of a filtration with filters of 0.22 µm of pore diameter took place, followed by freezing at -80 °C and finally freeze-drying. This last process allowed to have an enriched sample of the substance with which to do the subsequent tests.

### 4.2.2. Analysts of the capacity of the supernatant to inhibit the proliferation of rotavirus SA-11 in the cellular line MA-104

As in previous studies using intact cells, this assay was made following two strategies:
Strategy A - infection of MA-1 04 cells with rotavirus SA-11, previously incubated with the supernatant obtained in the previous section.
Strategy B - incubation of the cells with the supernatant and subsequent infection with rotavirus SA-11.

The protocol followed for the infection began with the formation of a monolayer of cells by means of seeding of MA-104 cells in MEM medium on 96-well plate (1.5x10⁵ cells/ml). The plates were incubated during 24 h at 37 °C in atmosphere with 5% (v/v) of CO₂. At the same time the activation of rotavirus was carried out (inoculum of 2x10⁸ ffu) by means of the incubation with type IX trypsin at 10 µg/ml during 30 min at 37 °C.

Strategy A: a suitable dilution of the virus was mixed with the different aliquots of supernatant and it was incubated at 37 °C during 1 h. The mixture virus/supernatant was inoculated (100 µl/well) in the monolayers of MA-1 04 cells and it was incubated at 37 °C during 1 h with 5% (v/v) of CO₂. After the adsorption period, the virus inoculums were removed and the cells were washed with PBS buffer. MEM medium was added to the cells (100 µl/well) without serum and finally, it was incubated 15-18 h at 37 °C with 5% (v/v) of CO₂. After the incubation, the infected cell cultures were tested by the immunoperoxidase focus detection assay evaluating the viral replication inhibitory capacity by means of the calculation of reduction of infectious foci.

Strategy B: supernatant aliquots were mixed with MA-104 cells and they were incubated 1 h at 37 °C. Then, the supernatant were extracted from the wells and cells were infected with a suitable dilution of rotavirus SA-11, previously activated. This was incubated at 37 °C during 1 h with 5% (v/v) of CO₂. After the adsorption period, the virus inoculums were removed and the cells were washed with PBS buffer. MEM medium was added to the cells (100 µl/well) without serum and finally, it was incubated 15-18 h at 37 °C with 5% (v/v) of CO₂. After the incubation, the infected cell cultures were tested with the immunoperoxidase focus detection assay evaluating the viral replication inhibitory capacity by means of the calculation of reduction of infectious foci.

In strategy A, the CECT 7210 strain produced a reduction of infectious foci of a 51.6% (1.0x10⁷ ffu/ml virus). In strategy B, the percentage of reduction was 83% (2.53x10⁶ ffu/ml virus). As in the previous study using the intact microbial cells, also in this case the preliminary interaction between the microbial supernatant and the mammalian cells were more effective. These facts stronly suggest that interactions between the surface of the eukaryotic cell and the supernatant are important factors for the inhibition of virus replication.

In conclusion, these two studies show that the supernatant of CECT 7210 strain produced inhibition of the infection by rotavirus, being the inhibition greater when the supernatant aliquots were previously incubated with MA-104 cells.

### 5.1. Characterization of the substance of interest. Processing of the supernatant with proteases

In order to verify the possible protein nature of the substance of interest, the freeze-dried samples of 25 ml of supernatant were treated with the proteases proteinase K and pepsin. The freeze-dried sample treated with pepsin was re-suspended in 1.25 ml of McIIvaine buffer 50 mM pH 4.0, to which 0.109 ml of a pepsin solution was added (50 mg/ml). This mixture was incubated at 37 °C during 2 h, and in order to stop the reaction, pepsin was inactivated taking the solution to pH 6.5 with the addition of 1.14 ml of sodium phosphate buffer 50 mM pH 8. The freeze-dried sample treated with proteinase K was re-suspended in 2.4 ml of phosphate buffer 50 mM pH 6.5, to which 0.1 ml of a proteinase K solution was added (50 mg/ml). This mixture was incubated at 37 °C during 2 h, and in order to stop the reaction, proteinase K was inactivated incubating the solution at 100 °C during 15 min.

Analysis of the inhibition of the proliferation of rotavirus SA-11 for the aliquots processed by proteases, was perfomed as explained in the previous section, following the two strategies. Results are shown in TABLES 3 and 4.

**TABLE 3. Virus samples and aliquots incubated 1 h at 37 °C with Strategy A.**

| Sample | ffu/ml virus | % infection |
|---|---|---|
| Pepsin | 2.9x10⁷ | 134.6 |
| Proteinase K | 2.5x10⁷ | 117.9 |
| Negative control QC3 | 2.6x10⁷ | 121.7 |
| Assay positive control | 2.1x10⁷ | 100.0 |
| **CECT 7210 strain supernatant** | **1.0x10⁷** | **48.4** |
| Negative control C1 | 2.1x10⁷ | 100.0 |

**TABLE 4. Cells samples and aliquots incubated 1 h at 37 °C with Strategy B.**

| Sample | ffu/ml virus | % infection |
|---|---|---|
| Pepsin | 1.76x10⁷ | 117.9 |
| Proteinase K | 1.52x10⁷ | 101:8 |
| Negative control QC3 | 1.44x10⁷ | 96.4 |
| Assay positive control | 1.49x10⁷ | 100.0 |
| **CECT 7210 strain supernatant** | **2.53x10⁶** | **17.0** |
| Negative control C1 | 1.49x10⁷ | 100.0 |

The volume of all the samples in TABLES 3 and 4 was 2.5 ml obtained by concentrating ten times the solution with respect to the initial volume of supernatant. As tested sample of inhibition, a freeze-dried sample (CECT 7210 strain supernatant) no treated with proteases was used. In this case, the freeze-dried sample of 25 ml was re-suspended in 2.5 ml of sodium phosphate buffer 50 mM pH 6.5.

As negative control of inhibition, a freeze-dried sample no fermented was used. In this case, the freeze-dried sample of 25 ml of culture medium was re-suspended in 2.5 ml of sodium phosphate buffer 50 mM pH 6.5. The negative controls included in both tests were with supernatant of culture medium MRS-C without inoculating (C1) and with this same supernatant but incubated like the rest of the samples during 2 h at 37 °C (QC3).

As positive control of infection, a solution composed by cells and viruses was used.

As shown in TABLES 3 and 4, treatment with different proteases gives rise to the loss of the inhibition capacity, conforming the protein nature of the substance/s responsible of the inhibition of the infection by rotavirus. These results were confirmed by similar treatments with lipases that they did not give reduction of the inhibiting capacity.

### 5.2. Purification and identification of the substance/s of interest by cationic exchange, chromatoaraphy followed by inverse phase chromatography

620 ml of the CECT 7210 strain supernatant was applied to a cationic exchange column (HiPrep 16/10 SP FF). All those cationic nature proteins which were adsorbed to the resin of the column were eluted with buffer equilibrated with NaCl 1 M without gradient. Thus, all the proteins were eluted simultaneously in few fractions (fractions 2, 3 and 4) and in a minimum volume, so that the substance of interest was concentrated. The volume of each collected fraction was 10 ml. Each fraction collected by cationic exchange was subjected to an ultrafiltration process using 5000 Da filters (Amicon Ultra, Millipore). Thus, the filtered volume contained those proteins with a molecular weight lower than 5000 Da, and the retained volume kept those proteins with an upper molecular weight.

The filtered volumes obtained with fractions 2, 3 and 4 from cationic exchange were subjected to another stage of inverse phase chromatography to purify, and later to identify by mass spectrometry, that substance/s that provides the inhibition of the infection by rotavirus. For the inverse phase chromatography, a RESOURCE RPC 3 ml column was used, working in gradient with the eluents A) 5% 2-propanol, 0.1% trifluoroacetic (TFA) in distilled water and B) 0.1% TFA in 2-propanol. In this chromatography fractions of 2 ml were obtained. An aliquot of these 2 ml was taken to dryness to eliminate the dissolvent and was re-suspended in a volume of MEM buffer just before beginning the test of inhibition of rotavirus with cell cultures.

The selected fractions to test the inhibitory capacity of the infection by rotavirus from fraction 2 of cationic exchange were fractions of inverse phase 2/5, 2/6, 2/7, 2/8, 2/9, 2/13, 2/14, 2/18, 2/20 and 2/23. From fraction 3 of cationic exchange, the following fractions were selected from inverse phase: 3/7, 3/8, 3/9, 3/10, 3/11, 3/12, 3/13, 3/16, 3/17, 3/19, 3/20 and 3/21, and from fraction 4 of cationic exchange, fractions 4/5, 4/6, 4/7, 4/8, 4/11 and 4/27 of inverse phase were selected.

**TABLE 5. Inhibition of rotavirus infection by inverse phase fractions obtained from fraction 2 of cationic exchange (previous incubation of cells with aliquots 1 h at 37 °C (Strategy B)**

| Sample | ffu/ml virus | % infection |
|---|---|---|
| 2/5 | 1.65x10⁷ | 199.6 |
| 2/6 | 9.00x10⁶ | 108.9 |
| 2/7 | 9.30x10⁶ | 112.5 |
| 2/8 | 9.10x10⁶ | 110.1 |
| 2/9 | 8.40x10⁶ | 101.6 |
| **2/13** | **4.50x10⁶** | **54.4** |
| 2/14 | 1.21x10⁷ | 146.4 |
| 2/18 | 8.60x10⁶ | 104.0 |
| 2/20 | 1.24x10⁷ | 150.0 |
| 2/23 | 8.40x10⁶ | 101.6 |
| Control | 8.27x10⁶ | 100.0 |

**TABLE 6. Inhibition of rotavirus infection by inverse phase fractions obtained from fraction 3 of cationic exchange (previous incubation of cells with aliquots 1 h at 37 °C (Strategy B)**

| Sample | ffu/ml virus | % infection |
|---|---|---|
| 3/7 | 2.07x10⁷ | 143.8 |
| 3/8 | 1.84x10⁷ | 127.8 |
| **3/9** | **8.20x10⁶** | **56.9** |
| **3/10** | **3.40x10⁶** | **23.6** |
| 3/11 | 1.64x10⁷ | 113.9 |
| 3/12 | 1.80x10⁷ | 125.0 |
| 3/13 | 2.21x10⁷ | 153.5 |
| 3/16 | 2.18x10⁷ | 151.4 |
| 3/17 | 2.04x10⁷ | 141.7 |
| 3/19 | 1.68x10⁷ | 116.7 |
| 3/20 | 1.65x10⁷ | 114.6 |
| 3/21 | 1.79x10⁷ | 124.3 |
| Control | 1.44x10⁶ | 100.0 |

**TABLE 7. Inhibition of rotavirus infection by inverse phase fractions obtained from fraction 4 of cationic exchange (previous incubation of cells with aliquots 1 h at 37 °C (Strategy B)**

| Sample | ffu/ml virus | % infection |
|---|---|---|
| **4/5** | **9.40x10⁶** | **72.9** |
| 4/6 | 1.48x10⁷ | 114.7 |
| 4/7 | 1.41x10⁷ | 109.6 |
| **4/8** | **3.33x10⁶** | **25.8** |
| **4/11** | **3.60x10⁶** | **27.9** |
| **4/27** | **9.10x10⁶** | **70.5** |
| Control | 1.29x10⁷ | 100.0 |

The fractions that gave a positive result of inhibition were analyzed by MALDI to determine the molecular weights of the peptide/s present in each fraction. Analyzing all the spectra, the only signal present in all the selected fractions corresponded to a peptide with a molecular weight of 1282.63 Da. Thus, one of the fractions was determined by sequencing, and the result was a peptide of 11 amino acids whose sequence was MHQPHQPLPPT and whose molecular mass was 1282.63 Da. This sequence was sent against the data base of proteins/peptides by means of the BLAST algorithm and it was observed that this sequence was part of the cow milk β-casein. The analysis of all the experimental results leads to think that CECT 7210 strain does not produce this peptide. Instead of this, the peptide should be the result of the digestion of the cow milk β-casein by a protease segregated by the CECT 7210 strain. A peptide of 13 amino acids and molecular weight of 1513 Da, was also present in most of the fractions.

### 6. In vivo protection against rotavirus

### 6.1. Studies of the capacity of colonization of the mouse intestinal tract and its implication in the in vivo protection against infections with rotavirus

Cultures of CECT 7210 strain bacteria were grown during 24 h at 37 °C in anaerobic conditions. From these cultures, inocula at 1% (w/v) were made in 25 ml of MRS-C medium. After 17 h of incubation in identical conditions, they were washed twice in saline solution 0.09% to an inoculum of 1x10⁹ cfu/ml.

The bacteria were resuspended in sodium bicarbonate buffer and frozen at - 70 °C in glycerol 20% until their use.

Two groups of nine BALB/c mice of 8 weeks of age were assayed. Group 1 was the control group and group 2 was treated with CECT 7210 strain. The control group was treated in each dose with 100 µl/mouse of sodium bicarbonate. The second group was treated in each dose with 10⁹ cfu/mouse of CECT 7210 strain resuspended in 100 µl of sodium bicarbonate too. All the tests were made at different times in each of the groups. The inoculation scheme that was followed was:

**TABLE 8. (¹ hours from the inoculation of the 1^{st} dose of CECT 7210 strain)**

| Day | CECT 7210 strain dose | Feces collection |
|---|---|---|
| 1 | 1^{st} dose | 4 hours¹ and 10 hours |
| 2 | 2^{nd} dose | 24 hours¹ |
| 3 | 3^{rd} dose | 48 hours¹ |
| 4 | -- | -- |
| 5 | - | 96 hours¹ |
| 6 | -- | -- |
| 7 | -- | -- |
| 8 | -- | 168 hours¹ |

Before the administration to mice, glycerol of the bacterial samples was eliminated by centrifugation at 4000 rpm during 5 min, and they were resuspended again in sodium bicarbonate buffer. Then, 100 µl of the suspension at a concentration of 10⁹ cfu/ml, were administered to each mouse with a catheter via gastric tract. To the mice of the control group 100 µl of sodium bicarbonate buffer were administered. Samples of feces were collected at different hours from the first CECT 7210 strain inoculation (see TABLE 8) and they were homogenized in PBS buffer (10% w/v). Then, they were shaken vigorously, centrifuged at 4000 rpm during 5 min, and stored at in refrigeration to be processed in less than 24 hours. The supernatant was frozen at - 30° C until its later use in the studies of the stimulation of the immune system.

The analysis of the viability of CECT 7210 strain in the gastrointestinal tract was made by means of seeding of the feces samples in selective medium. The medium used was a plate of selective medium for *Bifidobacterium* (BFM). Before the seeding, serial dilutions were made and several of these dilutions were plated by duplicate. The results showed that CECT 7210 strain reached their maximum viability at 10 h from the inoculum, with values at this point between 0.3 and 1x10⁹ cfu/ml very near to the 100% of viability. This time was considered as the suitable one to see possible effects of the probiotic activity and therefore it was used for the infection with rotavirus in later tests.

The determination of antibodies of IgA class in the faecal samples was made by ELISA. Polystyrene plates (Costar) were treated with 100 µl of 1/250 dilution of mouse anti-IgA sheep serum (Sigma) in carbonate/bicarbonate buffer pH 9.6, incubating them 2.5 hours at 37 °C and overnight at 4 °C until their use. Then, the supernatant was eliminated and washed three times with 200 µl of PBS buffer containing 0.1% of Tween 20 (v/v) (PBS-T). To each well, 100 µl of the samples supernatants diluted 1/1000 in PBS-T buffer containing 1% of BSA, were added. In order to detect the antibodies, mouse anti-IgA antibodies labelled with peroxidase were used at a dilution 1/2000 in PBS-T buffer with 1% BSA. Finally, 100 µl/well of OPD substrate (ortophenylenediamine) and H₂O₂ were added, and after 5 or 10 min of incubation to room temperature the reaction was stopped with 50 µl of H₂SO₄ 3M solution. The reading of absorbance was determined with an spectrophotometer at 492 nm. For the determination of the concentrations of IgA, a standard curve was prepared with serial dilutions of a solution of mouse IgA of well-known concentration.

The CECT 7210 strain produced an increase of the IgA concentration in feces. At long times from the first dose (96 h), animals treated with the CECT 7210 strain reached an IgA concentration of 30 mg/g in comparison with a value of 18 mg/g in the non-treated control group of animals.

### 6.2. Study of the capacity of protection of CECT 7210 strain in front of the infection with murine rotavirus McN strain

The same groups of mice of the previous study were used. After the first week, a new dose of CECT 7210 strain was administered to each mouse. This dose was repeated during 4 days more. Also, a sole oral inoculum of murine rotavirus McN strain containing 100 diarrhea-50 producing doses (DD₅₀) in 50 µl of balanced saline solution of Earle (EBSS). This inoculum was made 10 hours after the first CECT 7210 strain memory dose. Feces samples were taken during the nine following days to evaluate the presence of CECT 7210 strain by viable cells count, and the elimination of virus in feces by ELISA.

**TABLE 9. (¹ hours from the inoculation of the 1 rst dose of CECT 7210 strain;² inoculation of rotavirus 10 hours after the 1 st CECT 7210 strain memory dose; ³ hours from the inoculation of rotavirus)**

| Day | CECT 7210 strain dose and rotavirus infection | Feces collection | |
|---|---|---|---|
| 1 | 1^{st} memory dose Inoculation of rotavirus² | 10 hours¹ | |
| 2 | 2^{nd} dose | 24 hours³ | 34 hours¹ |
| 3 | 3^{rd} dose | 48 hours³ | 54 hours¹ |
| 4 | 4^{th} dose | 72 hours³ | |
| 5 | 5^{th} dose | 96 hours³ | 106 hours¹ |
| 6 | -- | 120 hours³ | |
| 7 | -- | 144 hours³ | |
| 8 | -- | 168 hours³ | |
| 9 | -- | 192 hours³ | |

For the microbial count after the infection, the samples were taken at 10, 34, 58, 106 and 168 hours after the first dose of CECT 7210 strain. The feces were resuspended in EBSS buffer 10%, homogenized with swab, shaken vigorously and centrifuged (4000 rpm 5 min). Pellet with CECT 7210 strain was collected and stored in refrigeration to be processed in less than 24 hours. The supernatant was frozen at - 30 °C until its later use in the studies of quantification of the viral antigen. Like in the previous section, the analysis of viability of CECT 7210 strain was made using plates of selective medium. Serial dilutions were made and several of these dilutions were plated by duplicate:

In order to evaluate the elimination of the virus in feces, ELISA protocol began with the treatment of the polystyrene 96-well plate with 100 µl of a dilution 1/500 of an anti-rotavirus sheep serum (Chemicon) in carbonate-bicarbonate buffer pH 9.6, incubating it 2.5 h at 37 °C and overnight at 4 °C. After three washings with PBS-T buffer, 100 µl of supernatant of feces samples were added, treated as described in the previous section, to a dilution 1/2 in PBS-T buffer with 1% BSA (w/v). After 1.5 h of incubation at 37° C, the supernatant was eliminated and washed four times with PBS-T buffer to add 100 µl /well of the monoclonal antibody anti-VP6 2F3E7. It was incubated again 1.5 h at 37° C and after four washings, 100 µl/well of a dilution 1/2000 of antibody anti-IgG of mouse conjugated with peroxidase in PBS-T-BSA buffer. After 1 h of incubation, it was washed four times with PBS-T and the solution of OPD and H₂O₂ was added. The reaction was stopped after 5-10 min with 50 µl of H₂SO₄ 3M solution. The reading of absorbance was determined at 492 nm. In order to quantify the virus concentration, serial dilutions of a titrated suspension of virus were analyzed, with the purpose of doing a standard curve and extrapolating the results of absorbance. The obtained results showed that the viable cells count was in its maximum of viability during the five days during CECT 7210 strain was provided and that this maximum settled down 10 hours after each dose of CECT 7210 strain.

In order to quantify the amount of viral antigen in feces, samples were taken during the nine days following the inoculation of the murine rotavirus McN according to the calendar. The detection of the antigen was made by ELISA. As positive control, the control group was inoculated with the same amount of rotavirus.

Regarding the evolution of the viral infection, the results indicated the existence of a delay in the proliferation of rotavirus the first 48 h after the infection (10⁶ ffu/ml in comparison with 4x10⁷ ffu/ml in the non-treated control group animals). This delay can be due to an initial reduction in the levels of replication of the rotavirus in the mice treated with CECT 7210 strain, although finally the virus infected all the groups of mice given the inoculated viral overdose. These differences are statistically significant.

### 7. Purification and identification of the CECT 7210 protease responsible of β-casein degradation and 11-mer production.

The CECT 7210 strain was grown in MRS (Pronadisa, Spain) with cysteine (0.05% p/v). After 17 hours at 37 °C, without stirring in anaerobic conditions the supernatant was obtained by centrifugation and the purification process began with the ammonium sulphate precipitation. After sample centrifugation (30 min, 10000 rpm) the obtained pellet was resuspended in Tris-HCl 20 mM pH 8.5 for the dialysis. 920 ml of the CECT 7210 supernatant obtained following the process of section 4.2.1, was applied to an anionic exchange column (HiPrep 16/10 Q XL) (GE Healthcare, Amersham Biosciences AB, Suecia). All those anionic nature proteins which were adsorbed to the resin of the column were eluted with buffer equilibrated (Tris-HCl 20 mM, pH 8.5, CaCl₂ 5 mM) with a 0-1 M NaCl gradient. Thus, all the proteins were eluted simultaneously in few fractions and in a minimum volume, so that the substance of interest was concentrated. Each fraction collected was subjected to an ultrafiltration process using 10000 Da filters (Extreme Amicon, Millipore). Thus, the filtered volume containing those proteins with a molecular weight higher than 10000 Da was subjected to another stage of gel filtration chromatography with a HiLoad Superdex 75 prep grade column (Amersham Bioscience, GE Healthcare) to purify, and later to identify by SDS/PAGE. The enzyme which was verified in each purification step had the protease activity.

The protease responsible of β-casein degradation was identified in a 12.5% SDS-PAGE. The molecular weight of the protease was observed around 34.8 and 80 kDa. The addition of 0.04 UI of protease activity contained in the last chromatographic purification fraction to β-casein (2%) as substrate in an enzymatic assay at 50 °C in citrate-phosphate buffer pH 6.4 during 48 hours, produced 0.0213 mg/ml of 11-mer peptide.

## Claims

1. A strain of *Bifidobacterium longum* biovar *infantis* deposited in the Colección Española de Cultivos Tipo (CECT) under the accession number CECT 7210, or a mutant thereof having an activity of inhibiting rotavirus and having the ability to produce a peptide from the fermentation of cow milk β-casein, said peptide having a length from 11 to 17 amino acids and an amino acid sequence which comprises SEQ ID NO: 1.

2. The strain according to claim 1, deposited in the Colección Espanola de Cultivos Tipo (CECT) under the accession number CECT 7210.

3. A bacterial preparation which comprises the strain as defined in any of the claims 1-2.

4. A food product which comprises a nutritionally effective amount of the strain as defined in any of the claims 1-2, together with appropriate amounts of other edible ingredients.

5. A nutritional composition which comprises a nutritionally effective amount of the strain as defined in any of the claims 1-2, together with appropriate amounts of other edible ingredients.

6. The composition according to claim 5, which is selected from the group consisting of a dietary supplement, an additive, and an infant formula.

7. A pharmaceutical composition which comprises a therapeutically effective amount of the strain as defined in any of the claims 1-2, together with appropriate amounts of pharmaceutical acceptable excipients and/or carriers.

8. The strain as defined in any of the claims 1-2, for use as a probiotic.

9. Use of the strain as defined in any of the claims 1-2, for the manufacture of a food product.

10. Use of the strain as defined in any of the claims 1-2, for the manufacture of a nutritional composition.

11. Use of the strain as defined in any of the claims 1-2, for the manufacture of a medicament for the therapeutic and/or preventive treatment of diarrhea in an animal including a human.

12. The use according to claim 11, wherein the diarrhea is caused by a rotavirus.

13. Use of the strain as defined in any of the claims 1-2, for the preparation of a peptide having a length from 11 to 17 amino acids and an amino acid sequence which comprises SEQ ID NO: 1, or a pharmaceutically acceptable salt thereof.

14. A process for the preparation of a peptide which has a length from 11 to 17 amino acids and an amino acid sequence which comprises SEQ ID NO: 1, or a pharmaceutically acceptable salt thereof, the process comprising fermenting cow milk β-casein which comprises the amino acid sequence SEQ ID NO: 1 with a strain as defined in any of the claims 1-2, and optionally isolating the peptide and/or converting them in a pharmaceutically acceptable salt.

## Patentansprüche

1. Ein Stamm von *Bifidobacterium longum* biovar *infantis,* der bei der Colección Española de Cultivos Tipo (CECT) unter der Hinterlegungsnummer CECT 7210 hinterlegt ist, oder ein Mutant davon, der eine Rotavirus hemmende Aktivität hat und der die Fähigkeit hat, ein Peptid aus der Fermentation von Kuhmilch-β-Casein zu erzeugen, wobei das Peptid eine Länge von 11 bis 17 Aminosäuren hat und eine Aminosäuresequenz hat, die SEQ ID NO: 1 umfasst.

2. Der Stamm nach Anspruch 1, der bei der Colección Espanola de Cultivos Tipo (CECT) unter der Hinterlegungsnummer CECT 7210 hinterlegt ist.

3. Eine Bakterienzubereitung, die den Stamm wie in einem der Ansprüche 1-2 definiert umfasst.

4. Ein Lebensmittel umfassend eine ernährungsphysiologisch wirksame Menge des Stamms wie in einem der Ansprüche 1-2 definiert nebst angemessenen Mengen von weiteren essbaren Inhaltsstoffen.

5. Eine ernährende Zusammensetzung umfassend eine ernährungsphysiologisch wirksame Menge des Stamms wie in einem der Ansprüche 1-2 definiert nebst angemessenen Mengen von weiteren essbaren Inhaltsstoffen.

6. Die Zusammensetzung nach Anspruch 5, die ausgewählt ist aus der Gruppe bestehend aus einem Nahrungsergänzungsmittel, einen Zusatzstoff, und eine Säuglingsnahrung.

7. Eine pharmazeutische Zusammensetzung, die eine therapeutisch wirksame Menge des Stamms wie in einem der Ansprüche 1-2 definiert nebst angemessenen Mengen von pharmazeutisch akzeptablen Hilfsstoffen und/oder Trägerstoffen umfasst.

8. Der Stamm wie in einem der Ansprüche 1-2 definiert zur Verwendung als probiotisches Mittel.

9. Verwendung des Stamms wie in einem der Ansprüche 1-2 definiert zur Herstellung eines Lebensmittels.

10. Verwendung des Stamms wie in einem der Ansprüche 1-2 definiert zur Herstellung einer Nährstoffzusammensetzung.

11. Verwendung des Stamms wie in einem der Ansprüche 1-2 zur Herstellung eines Arzneimittels für die therapeutische und/oder vorbeugende Behandlung von Durchfall bei einem Tier, einschließlich des Menschen.

12. Die Verwendung nach Anspruch 11, wobei der Durchfall durch einen Rotavirus verursacht wird.

13. Verwendung eines Stamms wie in einem der Ansprüche 1-2 definiert für die Vorbereitung eines Peptids, das eine Länge von 11 bis 17 Aminosäuren hat und eine Aminosäuresequenz hat, die SEQ ID NO: 1 umfasst, oder eines pharmazeutisch akzeptablen Salzes davon.

14. Verfahren zur Herstellung eines Peptids, das eine Länge von 11 bis 17 Aminosäuren hat und eine Aminosäuresequenz hat, die SEQ ID NO: 1 umfasst, oder eines pharmazeutisch akzeptablen Salzes davon, wobei das Verfahren die Fermentation von Kuhmilch-β-Casein, das die Aminosäuresequenz SEQ ID NO: 1 umfasst mit einem Stamm wie in einem der Ansprüche 1-2 definiert umfasst, und wahlweise die Isolierung des Peptids und/oder die Umsetzung deren in ein pharmazeutisch akzeptables Salz.

## Revendications

1. Une souche de *Bifidobacterium longum* biovar *infantis* déposée à la Colección Española de Cultivos Tipo (CECT) sous le numéro de dépôt CECT 7210 ou un mutant de celle-ci ayant une activité d'inhibition du rotavirus et ayant la capacité de produire un peptide à partir de la fermentation de la β-caséine du lait de vache, ayant ledit peptide une longueur allant de 11 à 17 acides aminés et une séquence d'acides aminés qui comprend la SEQ ID NO: 1.

2. La souche selon la revendication 1, déposée à la Colección Espanola de Cultivos Tipo (CECT) sous le numéro de dépôt CECT 7210.

3. Une préparation bactérienne qui comprend la souche telle que définie dans l'une quelconque des revendications 1-2.

4. Un produit alimentaire qui comprend une quantité nutritionnellement effective de la souche telle que définie dans l'une quelconque des revendications 1-2, avec des quantités appropriées d'autres ingrédients comestibles.

5. Une composition nutritionnelle qui comprend une quantité nutritionnellement effective de la souche telle que définie dans l'une quelconque des revendications 1-2, avec des quantités appropriées d'autres ingrédients comestibles.

6. La composition selon la revendication 5, qui est choisie dans le groupe constitué d'un supplément alimentaire, d'un additif, et d'une préparation pour nourrissons.

7. Une composition pharmaceutique qui comprend une quantité thérapeutiquement effective de la souche telle que définie dans l'une quelconque des revendications 1-2, avec des quantités appropriées d'excipients et/ou de véhicules pharmaceutiquement acceptables.

8. La souche telle que définie dans l'une quelconque des revendications 1-2 pour son utilisation en tant que matériau probiotique.

9. L'utilisation de la souche telle que définie dans l'une quelconque des revendications 1-2 pour la fabrication d'un produit alimentaire.

10. L'utilisation de la souche telle que définie dans l'une quelconque des revendications 1-2 pour la fabrication d'une composition nutritionnelle.

11. L'utilisation de la souche telle que définie dans l'une quelconque des revendications 1-2 pour la fabrication d'un médicament pour le traitement thérapeutique et/ou préventif de la diarrhée chez un animal, y compris les humains.

12. L'utilisation selon la revendication 11, dans laquelle la diarrhée est causée par un rotavirus.

13. Utilisation de la souche telle que définie dans l'une quelconque des revendications 1-2, pour la préparation d'un peptide ayant une longueur allant de 11 à 17 acides aminés et un séquence d'acides aminés qui comprend la SEQ ID NO: 1, ou un sel pharmaceutiquement acceptable de celui-ci.

14. Un procédé pour la préparation d'un peptide qui a une longueur allant de 11 à 17 acides aminés et une séquence d'acides aminés qui comprend la SEQ ID NO: 1, ou un sel pharmaceutiquement acceptable de celui-ci, comprenant le procédé la fermentation de β-caféine du lait de vache qui comprend la séquence d'acides aminés SEQ ID NO: 1 avec une souche telle que définie dans l'une quelconque des revendications 1-2, et facultativement isoler le peptide et/ou sa conversion dans un sel pharmaceutiquement acceptable.


### SEQUENCE LISTING

<110> Laboratorios Ordesa, S.L.
<120> A novel strain of Bifidobacterium and active peptides against rotavirus infections
<130> Ordesa P896PC00 probiotic
<150> EP07111189.2
   <151> 2007-06-27
<160> 19
<170> PatentIn version 3.5
<210> 1
   <211> 11
   <212> PRT
   <213> Artificial
<220>
   <223> peptide from supernatant
<400> 1
   Met His Gln Pro His Gln Pro Leu Pro Pro Thr
   1 5 10
<210> 2
   <211> 12
   <212> PRT
   <213> Artificial
<220>
   <223> peptide from supernatant
<400> 2
   Met His Gln Pro His Gln Pro Leu Pro Pro Thr Val
   1 5 10
<210> 3
   <211> 13
   <212> PRT
   <213> Artificial
<220>
   <223> peptide from supernatant
<400> 3
   Met His Gln Pro His Gln Pro Leu Pro Pro Thr Val Met
   1 5 10
<210> 4
   <211> 14
   <212> PRT
   <213> Artificial
<220>
   <223> peptide from supernatant
<400> 4
   Met His Gln Pro His Gln Pro Leu Pro Pro Thr Val Met Phe
   1 5 10
<210> 5
   <211> 12
   <212> PRT
   <213> Artificial
<220>
   <223> peptide from supernatant
<400> 5
   Trp Met His Gln Pro His Gln Pro Leu Pro Pro Thr
   1               5                   10
<210> 6
   <211> 13
   <212> PRT
   <213> Artificial
<220>
   <223> peptide from supernatant
<400> 6
   Trp Met His Gln Pro His Gln Pro Leu Pro Pro Thr Val
   1               5                   10
<210> 7
   <211> 14
   <212> PRT
   <213> Artificial
<220>
   <223> peptide from supernatant
<400> 7
   Trp Met His Gln Pro His Gln Pro Leu Pro Pro Thr Val Met
   1               5                   10
<210> 8
   <211> 15
   <212> PRT
   <213> Artificial
<220>
   <223> peptide from supernatant
<400> 8
   Trp Met His Gln Pro His Gln Pro Leu Pro Pro Thr Val Met Phe
   1               5                   10                  15
<210> 9
   <211> 13
   <212> PRT
   <213> Artificial
<220>
   <223> peptide from supernatant
<400> 9
   Ser Trp Met His Gln Pro His Gln Pro Leu Pro Pro Thr
   1               5                   10
<210> 10
   <211> 14
   <212> PRT
   <213> Artificial
<220>
   <223> peptide from supernatant
<400> 10
   Ser Trp Met His Gln Pro His Gln Pro Leu Pro Pro Thr Val
   1               5                   10
<210> 11
   <211> 15
   <212> PRT
   <213> Artificial
<220>
   <223> peptide from supernatant
<400> 11
   Ser Trp Met His Gln Pro His Gln Pro Leu Pro Pro Thr Val Met
   1               5                   10                  15
<210> 12
   <211> 16
   <212> PRT
   <213> Artificial
<220>
   <223> peptide from supernatant
<400> 12
   Ser Trp Met His Gln Pro His Gln Pro Leu Pro Pro Thr Val Met Phe
   1               5                   10                  15
<210> 13
   <211> 14
   <212> PRT
   <213> Artificial
<220>
   <223> peptide from supernatant
<400> 13
   Gln Ser Trp Met His Gln Pro His Gln Pro Leu Pro Pro Thr
   1 5 10
<210> 14
   <211> 15
   <212> PRT
   <213> Artificial
<220>
   <223> peptide from supernatant
<400> 14
   Gln Ser Trp Met His Gln Pro His Gln Pro Leu Pro Pro Thr Val
   1 5 10 15
<210> 15
   <211> 16
   <212> PRT
   <213> Artificial
<220>
   <223> peptide from supernatant
<400> 15
   Gln Ser Trp Met His Gln Pro His Gln Pro Leu Pro Pro Thr Val Met
   1 5 10 15
<210> 16
   <211> 17
   <212> PRT
   <213> Artificial
<220>
   <223> peptide from supernatant
<400> 16
   Gln Ser Trp Met His Gln Pro His Gln Pro Leu Pro Pro Thr Val Met
   1 5 10 15
   Phe
<210> 17
   <211> 20
   <212> DNA
   <213> Artificial
<220>
   <223> primer 616V for 16S rRNA gene
<400> 17
   agagtttgat ymtggctcag 20
<210> 18
   <211> 15
   <212> DNA
   <213> Artificial
<220>
   <223> primer 699R for 16S rRNA gene
<400> 18
   rgggttgcgc tcgtt 15
<210> 19
   <211> 1166
   <212> DD1A
   <213> Artificial
<220>
   <223> sequence 16S rRNA
<220>
   <221> misc feature
   <222> (792)...(792)
   <223> n is a, c, g, or t
<220>
   <221> misc feature
   <222> (864)..(864)
   <223> n is a, c, g, or t
<220>
   <221> misc feature
   <222> (885)..(885)
   <223> n is a, c, g, or t
<220>
   <221> misc_feature
   <222> (950)..(950)
   <223> n is a, c, g, or t
<220>
   <221> misc feature
   <222> (961)..(961)
   <223> n is a, c, g, or t
<220>
   <221> misc_feature
   <222> (993)..(993)
   <223> n is a, c, g, or t
<220>
   <221> misc_feature
   <222> (1006) .. (1006)
   <223> n is a, c, g, or t
<220>
   <221> misc_feature
   <222> (1022)..(1022)
   <223> n is a, c, g, or t
<400> 19
   cgaactgaga ccggttttca gggatccgct ccgcgtcgcc gcgtcgcatc ccgttgtacc        60
   ggccattgta gcatgcgtga agccctggac gtaaggggca tgatgatctg acgtcatccc       120
   caccttcctc cgagttaacc ccggcggtcc cccgtgagtt cccggcataa tccgctggca       180
   acacggggcg agggttgcgc tcgttgcggg acttaaccca acatctcacg acacgagctg       240
   acgacgacca tgcaccacct gtgaacccgc cccgaaggga agccgtatct ctacgaccgt       300
   cgggaacatg tcaagcccag gtaaggttct tcgcgttgca tcgaattaat ccgcatgctc       360
   cgccgcttgt gcgggccccc gtcaatttct ttgagtttta gccttgcggc cgtactcccc       420
   aggcgggatg cttaacgcgt tagctccgac acggaacccg tggaacgggc cccacatcca       480
   gcatccaccg tttacggcgt ggactaccag ggtatctaat cctgttcgct ccccacgctt       540
   tcgctcctca gcgtcagtaa cggcccagag acctgccttc gccattggtg ttcttcccga       600
   tatctacaca ttccaccgtt acaccgggaa ttccagtctc ccctaccgca ctcaagcccg       660
   cccgtacccg gcgcggatcc accgttaagc gatggacttt cacaccggac gcgacgaacc       720
   gcctacgagc cctttacgcc caataattcc ggataacgct tgcaccctac gtattaccgc       780
   ggctgctggc angtagttag ccggtgctta ttcaacgggt aaactcactc tcgcttgctc       840
   cccgataaaa gaggtttaca accngaaggc ctccatccct cacgnggggt cgctgcatca       900
   ggcttgcgcc cattgtgcaa tattccccac tgctgcctcc cgtaggagtn tgggccgtat       960
   ntcagtccca atgtggccgg tcgccctctc agnccggcta cccgtngaag ccacggtggg      1020
   cngttacccc gccgtcaagc tgataggacg cgaccccatc ccataccgcg aaagctttcc      1080
   cagaagacca tgcgatcaac tggagcatcc ggcattacca cccgtttcca ggagctattc      1140
   cggtgtatgg ggcaggtcgg tcacgc                                           1166